(19) Europäisches Patentamt
European Patent Office
Office européen des brevets

(11) Veröffentlichungsnummer: 0 014 372
A1

(12) # EUROPÄISCHE PATENTANMELDUNG

(21) Anmeldenummer: 80100308.8

(22) Anmeldetag: 22.01.80

(51) Int. Cl.³: C 07 D 311/56
A 01 N 43/16

(30) Priorität: 23.01.79 CH 658/79
06.09.79 CH 8043/79

(43) Veröffentlichungstag der Anmeldung:
20.08.80 Patentblatt 80/17

(84) Benannte Vertragsstaaten:
CH DE FR GB IT

(71) Anmelder: CIBA-GEIGY AG
Patentabteilung Postfach
CH-4002 Basel(CH)

(72) Erfinder: Beriger, Ernst, Dr.
Grabenmattweg 29
CH-4123 Allschwil(CH)

(74) Vertreter: Zumstein sen., Fritz, Dr. et al,
Bräuhausstrasse 4
D-8000 München 2(DE)

(54) Ammoniumsalze von 4-Hydroxy-3-(4-trifluormethylphenylcarbamoyl)-cumarin, Verfahren zu ihrer Herstellung, Mittel welche diese Salze enthalten und deren Verwendung zur Bekämpfung von Insekten.

(57) Ammoniumsalze der Formel I

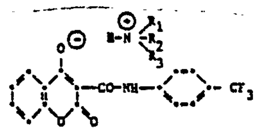

worin $R_1$, $R_2$ und $R_3$ je eine gegebenenfalls hydroxysubstituierte $C_1$-$C_6$-Alkylgruppe oder eine $C_3$-$C_6$-Alkenyl- oder $C_3$-$C_6$-Alkinylgruppe bedeuten, Verfahren zu ihrer Herstellung, Mittel welche diese Salze als aktive Komponente enthalten und deren Verwendung zur Bekämpfung von Insekten.

EP 0 014 372 A1

CIBA-GEIGY AG
Basel (Schweiz)

5-12196/1+2/+

# Ammoniumsalze von 4-Hydroxy-3-(4-trifluormethylphenylcarbamoyl)-cumarin, Verfahren zu ihrer Herstellung, Mittel welche diese Salze enthalten und deren Verwendung zur Bekämpfung von Insekten

Die vorliegende Erfindung betrifft neue Ammoniumsalze von 4-Hydroxy-3-(4-trifluormethylphenylcarbamoyl)-cumarin, welche eine Wirkung gegen Insekten besitzen, und Verfahren zu ihrer Herstellung sowie Schädlingsbekämpfungsmittel, welche die genannten Salze als aktive Komponente enthalten, und Verfahren zur Bekämpfung von Schädlingen unter Verwendung der neuen Verbindungen.

4-Hydroxy-3-(4-trifluormethylphenylcarbamoyl)-cumarin ist als eine insektizid-wirksame Substanz aus der DOS 2,643,476 bereits bekannt. Nach vorliegender Erfindung werden neuartige Ammoniumsalze derselben Verbindung bereitgestellt, welche eine verglichen mit der bekannten freien Verbindung wesentlich erhöhte Wirkung gegen Insekten, vor allem gegen pflanzenschädigende Insekten aufweisen.

Die erfindungsgemässen neuen Ammoniumsalze von 4-Hydroxy-3-(4-trifluormethylphenylcarbamoyl)-cumarin entsprechen der Formel I

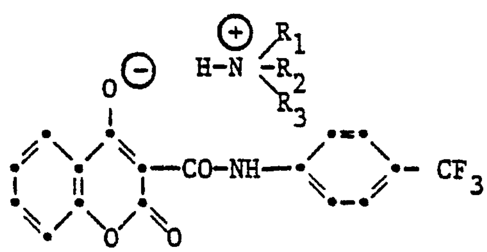

(I)

worin $R_1$, $R_2$ und $R_3$ unabhängig voneinander eine gegebenenfalls hydroxy-substiuierte $C_1$-$C_6$ -Alkylgruppe oder eine $C_3$-$C_6$ Alkenyl- oder $C_3$-$C_6$-Alkinylgruppe bedeuten.

Eine bevorzugte Untergruppe bilden die Verbindungen der Formel I, worin $R_1$, $R_2$ und $R_3$ unabhängig voneinander eine $C_1$-$C_6$-Alkyl-, 2-Hydroxyäthyl-, 2-Propenyl- oder 2-Propinylgruppe bedeuten.

Eine weitere bevorzugte Untergruppe bilden die Verbindungen der Formel I, worin $R_1$ eine $C_1$-$C_6$-Alkyl-, 2-Hydroxyäthyl-, 2-Propenyl- oder 2-Propinylgruppe, insbesondere eine $C_1$-$C_4$-Alkylgruppe bedeutet und $R_2$ und $R_3$ unabhängig voneinander eine $C_1$-$C_4$-Alkylgruppe bedeuten.

Die erfindungsgemässen Verbindungen der Formel I werden vorzugsweise in Form eines kristallinen Festkörpers hergestellt und verwendet.

Nach vorliegender Erfindung wurde nun festgestellt, dass die Ammoniumsalze der oben beanspruchten Formel I eine stark ausgeprägte Wirksamkeit gegen Insekten, insbesondere pflanzenschädigende Insekten, verschiedener Ordnungen (wie z.B. Lepidoptera, Coleoptera, Homoptera, Heteroptera, Diptera, Thysanoptera, Orthoptera und Hymenoptera) aufweisen. Darüber hinaus wurde weiter gefunden, dass im Feldversuch die genannten Salze gegenüber dem bekannten freien 4-Hydroxy-3-(4-trifluormethylphenylcarbamoyl)-cumarin eine über einen längeren Zeitraum überraschenderweise überlegene Aktivität gegen Insekten der Ordnung Coleoptera und vor allem der Familien Curculionidae und Chrysomellidae besitzen. Betreffend die Wirksamkeit gegen Vertreter der Familie Curculionidae ist die hervorragende Dauer-Wirkung der erfindungsgemässen Salze gegen Insekten der Spezies Anthonomus grandis hervorzuheben. Die Verbindungen der Formel I sind demzufolge zur Bekämpfung von pflanzenparasitären Insekten in Kulturen von Nutz- und Zierpflanzen, vor allem in Baumwollkulturen besonderes geeignet.

Darüber hinaus besitzen die genannten Salze ebenfalls eine gute Wirkung gegen ektoparasitäre Insekten (wie z.B. Lucilia sericata) sowie gegen Insekten, welche in den Gebieten Hygiene und Vorratsschutz schädlich sind (wie z.B. Musca domestica und Sitophilus granarinus).

Die Ammoniumsalze der Formel I werden analog bekannten Verfahren hergestellt, indem man z.B. das 4-Hydroxy-3-(4-trifluormethylphenyl-carbamoyl)-cumarin der Formel II

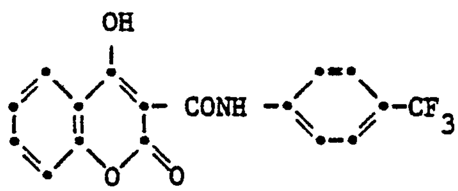

(II)

mit einem Amin der Formel III

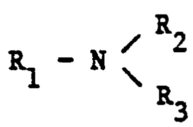

(III)

reagieren lässt, wobei in der Formel III $R_1$, $R_2$ und $R_3$ die für Formel I bereits angegebenen Bedeutungen haben. Das Produkt (Verbindung der Formel I) wird vorzugsweise durch übliche Trennungsmethoden in Form eines kristallinen Festkörpers aus dem entstandenen Reaktionsgemisch isoliert.

Das Verfahren wird zweckmässig bei einer Temperatur zwischen 0° und 100°C, insbesondere 20° und 80°C, bei normalem Druck und vorzugsweise in Gegenwart eines gegenüber den Reaktionsteilnehmern inerten Lösungs- oder Verdünnungsmittels durchgeführt. Als Lösungs- oder Verdünnungsmittel eignen sich z.B. Aether und ätherartige Verbindungen wie Diäthyläther, Diisopropyläther, Dioxan und Tetrahydrofuran; aromatische Kohlenwasserstoffe wie Benzol, Toluol und

Xylole; Ketone wie Aceton, Methyläthylketon und Cyclohexanon; und Alkohole wie Methanol, Aethanol und Methoxyäthanol.

Die in dem vorstehend aufgeführten Verfahren verwendeten Ausgangsstoffe sind bekannt (vgl. DOS 2,643,476).

Die Ammoniumsalze der Formel I werden erfindungsgemäss als solche verwendet oder bilden einen Bestandteil von Mitteln, welche noch geeignete Trägerstoffe oder Zuschlagstoffe oder Gemische solcher Stoffe enthalten.

Geeignete Träger und Zuschlagstoffe können fest oder flüssig sein und entsprechen den in der Formulierungstechnik üblichen Stoffen wie z.B. natürlichen oder regenerierten Stoffen, Lösungs-, Dispergier-, Netz-, Haft-, Verdickungs-, Binde- und/oder Düngemitteln.

Die insektizide Wirkung der erfindungsgemässen Mittel lässt sich durch Zusatz anderer Insektizide und/oder Akarizide wesentlich verbreitern.

Als Zusätze eignen sich z.B.: org. Phosphorverbindungen; Nitrophenole und deren Derivate; Formamidine; Harnstoffe; pyrethrinartige Verbindungen; Karbamate und chlorierte Kohlenwasserstoffe.

Die erfindungsgemässen Mittel können z.B. als Stäubemittel, Granulate, Dispersionen, Lösungen und Aufschlämmungen sowie als in Wasser dispergierbare Spritzpulver, Pasten, Emulsionen und Emulsionskonzentrate vorliegen und angewendet werden.

Der Gehalt an Wirkstoff (Ammoniumsalz der Formel I) in den oben beschriebenen Mitteln liegt zwischen 0,1 bis 95%, dabei ist zu erwähnen, dass bei der Applikation aus dem Flugzeug oder mittels

anderer geeigneter Applikationsgeräte auch höhere Konzentrationen eingesetzt werden können.

Die Wirkstoffe der Formel I können beispielsweise wie folgt formuliert werden:

Emulsionskonzentrat

| | |
|---|---|
| 5 bis max. 30 Gew.-Teile | Wirkstoff werden unter Rühren bei Zimmertemperatur in |
| 30 Gew.-Teilen | Dibutylphthalat |
| 10 Gew.-Teilen | Solvent 200 (niederviskoses hocharomat. Erdöldestillat) |
| 15 bis 35.Gew.-Teilen | Dutrex 238 FC (viskoses hocharomatisches Erdöldestillat) gelöst und mit |
| 10 Gew.-Teilen | eines Emulgatorgemisches, bestehend aus Ricinusöl-polyglykoläther und dem Calciumsalz der Dodecylbenzolsulfonsäure, versetzt. Das so erhaltene Emulsionskonzentrat gibt in Wasser milchige Emulsionen. |

Spritzpulver

| | |
|---|---|
| 5 bis 30 Gew.-Teile | des Wirkstoffes werden in einem Mischapparat mit |
| 5 Gew.-Teilen | eines aufsaugenden Trägermaterials (Kieselsäure K 320 oder Wessalon S) und |
| 55 bis 80 Gew.-Teilen | eines Trägermaterials (Bolus alba oder Kaolin B 24) und einem Dispergiermittelgemisch, bestehend aus |
| 5 Gew.-Teilen | eines Na-lauryl-sulfonates und |

| | |
|---|---|
| 5 Gew.-Teilen | eines Alkyl-aryl-polyglykoläthers, intensiv vermischt. Diese Mischung wird auf einer Stift- oder Luftstrahlmühle bis auf 5-15 um gemahlen. Das so erhaltene Spritzpulver gibt in Wasser eine gute Suspension. |

Pour-on-Lösung

| | |
|---|---|
| Wirksubstanz | 30,0 g |
| Natrium-dioctylsulfosuccinat | 3,0 g |
| Benzylalkohol | 48,0 g |
| Erdnussöl | 19,8 g |
| | 100,8 g = 100 ml |

Die Wirksubstanz wird in dem Benzylalkohol unter Rühren, eventuell auch unter leichtem Erwärmen, gelöst. Zu der Lösung wird das Natriumdioctylsulfosuccinat und das Erdnussöl gegeben und unter Erwärmen und gründlichem Durchmischen gelöst.

Die nachfolgenden Beispiele dienen zur näheren Erläuterung der Erfindung:

Beispiel 1: Herstellung des Triäthylammoniumsalzes von 4-Hydroxy-3-(4-trifluormethylphenylcarbamoyl)-cumarin

Zu einer Lösung von 17,5 g 4-Hydroxy-3-(4-trifluormethylphenylcarbamoyl)-cumarin in 100 ml abs. Aethanol wurden 11 g Triäthylamin bei Raumtemperatur zugegeben, wobei die Temperatur langsam auf 28°C stieg. Das erhaltene Reaktionsgemisch wurde während drei Stunden nachgerührt und die ausgeschiedenen Kristalle anschliessend abfiltriert. Auf diese Weise erhielt man das Triäthylaminsalz von 4-Hydroxy-3-(4-trifluormethylphenylcarbamoyl)-cumarin (Verbindung Nr. 1):

(1) 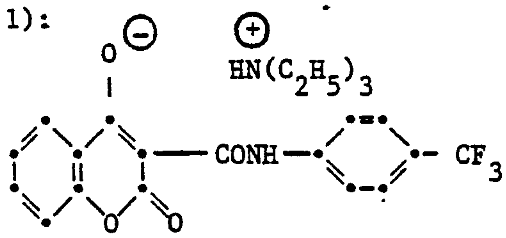

mit einem Smp. von 258°-260°C [ab 130°C leichte Veränderungen (Zersetzung) der Substanz ohne Schmelzen].

Die folgenden Verbindungen der Formel I können analog zu dem vorstehend beschriebenen Herstellungsverfahren hergestellt werden:

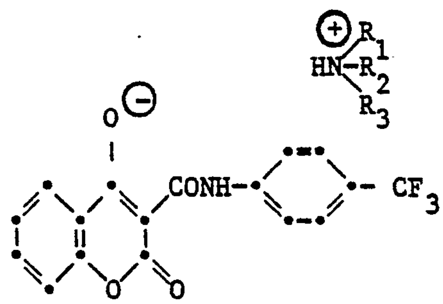

| Verb. Nr. | $R_1$ | $R_2$ | $R_3$ | physikalische Daten | |
|---|---|---|---|---|---|
| | | | | Zersetzung ab °C | Smp. °C |
| 2 | $CH_3$ | $CH_3$ | $CH_3$ | 80 | 256 |
| 3 | $n-C_3H_7$ | $n-C_3H_7$ | $n-C_3H_7$ | 190 | 258 |
| 4 | $i-C_3H_7$ | $i-C_3H_7$ | $i-C_3H_7$ | | |
| 5 | $n-C_4H_9$ | $n-C_4H_9$ | $n-C_4H_9$ | 200 | 258 |
| 6 | $n-C_6H_{13}$ | $CH_3$ | $CH_3$ | 149 | 256 |
| 7 | $HO-CH_2-CH_2-$ | $CH_3$ | $CH_3$ | 108 | 258 |
| 8 | $HO-CH_2-CH_2-$ | $C_2H_5$ | $C_2H_5$ | 130 | 257 |
| 9 | $CH_2=CH-CH_2-$ | $CH_3$ | $CH_3$ | 120 | 258 |
| 10 | $CH\equiv C-CH_2-$ | $CH_3$ | $CH_3$ | 130-137 | 258 |

Beispiel 2: Insektizide Frass- und Kontaktwirkung: Anthonomus grandis, Heliothis virescens und Spodoptera littoralis.

Baumwollpflanzen wurden mit einer wässrigen Emulsion enthaltend 0,05% der zu prüfenden Verbindung (erhalten aus einem 25%igen Spritzpulver) besprüht.
Nach dem Antrocknen des Belages wurden die Pflanzen mit Adulten bzw. Larven der Spezies

a) Anthonomus grandis (Adulte),
b) Heliothis virescens (Larven: L 3/L 4 Instar); oder
c) Spodoptera littoralis (Larven: L 3 Instar).

besiedelt. Man verwendete für jede Versuchsverbindung und für jede Test-Spezies zwei Pflanzen, und eine Auswertung der erzielten Abtötung erfolgte 2, 4, 24 und 48 Stunden nach Versuchsbeginn.

Der Versuch wurde bei 24°C und 60% relativer Luftfeuchtigkeit durchgeführt.

Salze gemäss Beispiel 1 zeigten im obigen Versuch eine gute Wirkung gegen Insekten der erwähnten Spezies.

Beispiel 3: Insektizide Frass- und Kontaktwirkung: Leptinotarsa decemlineata. (I)

Bei gleicher Arbeitsweise unter Verwendung von Kartoffelstauden anstelle von Baumwollpflanzen und Larven der Spezies Leptinotarsa decemlineata im L3-Stadium, wurde die im Beispiel 2 beschriebene Versuchsmethode wiederholt. Verbindungen gemäss Beispiel 1 zeigten ebenfalls in diesem Versuch eine gute Wirkung gegen Larven der Spezies Leptinotarsa decemlineata.

Beispiel 4: Insektizid Frass- und Kontakt-Dauerwirkung: Anthonomus grandis.

Unter Verwendung von Adulten der Spezies Anthonomus grandis wurde die im Beispiel 2 beschriebene Versuchsmethode wiederholt. Nur in diesem Fall wurden die Baumwollpflanzen erst 8 Tage nach Benetzung mit der Versuchs-Emulsion mit der Test-Spezies besiedelt. Die Auswertung der Mortalität wurde nach weiteren 24 und 48 Stunden durchgeführt.

Verbindungen gemäss Beispiel 1 zeigten im obigen Versuch eine gute Dauer-Wirkung gegen Insekten der Spezies Anthonomus grandis.

Beispiel 5: Wirkung gegen Leptinotarsa decemlineata II

Zwei 15 cm hohe Kartoffelstauden wurden mit 25 ml eines 0,05% Test-Substanz enthaltenden Aceton/Wasser-Gemisches (1:1) gespritzt.

Nach dem Antrocknen des Belages wurden die Kartoffelstauden mit je 10 Larven der Spezies Leptinotarsa decemlineata (L3-Stadium) besiedelt. Anschliessend wurde ein Plastikzylinder über die Pflanze gestülpt um eine eventuelle Abwanderung der Larven zu verhindern. Als Abschluss diente ein Kupfergazedeckel. Nach 2 Tagen wurde der Frass-Schaden bestimmt.

Verbindungen gemäss Beispiel 1 zeigten im obigen Versuch eine positive frasshemmende Wirkung (nur geringer Frass-Schaden festzustellen) gegen Insekten-Larven der Spezies Leptinotarsa decemlineata.

Beispiel 6 : Wirkung gegen Lucilia sericata

Zu 2 ml eines Zuchtmediums wurden 2 ml einer 0,1% Testsubstanz enthaltenden wässrigen Lösung gegeben. Nun wurden ca. 30 frisch geschlüpfte Lucilia sericata-Larven zum Zuchtmedium gegeben und nach 96 Stunden die insektizide Wirkung durch Ermittlung der Abtötungsrate festgestellt. Verbindungen gemäss Beispiel 1 zeigten im obigen Test eine gute Wirkung gegen Lucilia sericata-Larven.

Patentansprüche

1. Verbindungen der Formel I

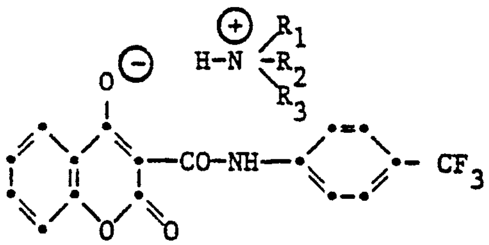

(I)

worin $R_1$, $R_2$ und $R_3$ unabhängig voneinander eine gegebenenfalls hydroxysubstituierte $C_1$-$C_6$-Alkylgruppe oder eine $C_3$-$C_6$-Alkenyl- oder $C_3$-$C_6$-Alkinylgruppe bedeuten.

2. Verbindungen nach Anspruch 1, worin $R_1$, $R_2$ und $R_3$ unabhängig voneinander eine $C_1$-$C_6$-Alkyl-, 2-Hydroxyäthyl-, 2-Propenyl- oder 2-Propinylgruppe bedeuten.

3. Verbindungen nach Anspruch 1 oder 2, worin $R_2$ und $R_3$ unabhängig voneinander eine $C_1$-$C_4$-Alkylgruppe bedeuten.

4. Verbindungen nach Anspruch 3, worin $R_1$ eine $C_1$-$C_4$-Alkylgruppe bedeutet.

5. Verbindung nach Anspruch 4, worin $R_1$, $R_2$ und $R_3$ je eine Methylgruppe bedeuten.

6. Verbindung nach Anspruch 4, worin $R_1$, $R_2$ und $R_3$ je eine Aethylgruppe bedeuten.

7. Verbindung nach Anspruch 4, worin $R_1$, $R_2$ und $R_3$ je eine n-Propylgruppe bedeuten.

8. Verbindung nach einem der Ansprüche 1 bis 7 in Form eines kristallinen Festkörpers.

9. Verfahren zur Herstellung von in einem der Ansprüche 1 bis 7 definierten Verbindungen, dadurch gekennzeichnet, dass man eine Verbindung der Formel II

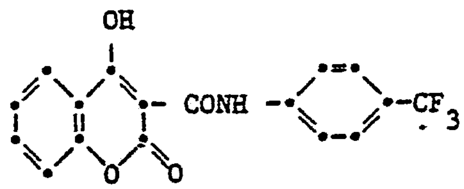

(II)

mit einem Amin der Formel III

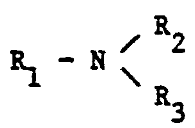

(III)

umsetzt, worin $R_1$, $R_2$ und $R_3$ die entsprechenden, in einem der Ansprüche 1 bis 7 angegebenen Bedeutungen haben.

10. Verfahren nach Anspruch 1, worin das Produkt in Form eines kristallinen Festkörpers aus dem erhaltenen Reaktionsgemisch gewonnen wird.

11. Mittel zur Bekämpfung von Insekten, dadurch gekennzeichnet, dass es als aktive Komponente eine der in einem der Ansprüche 1 bis 8 definierten Verbindungen enthält.

12. Verwendung von in einem der Ansprüche 1 bis 8 definierten Verbindungen zur Bekämpfung von Insekten.

0014372

Nummer der Anmeldung

EP 80 10 0308

Europäisches Patentamt

# EUROPÄISCHER RECHERCHENBERICHT

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| X | US - A - 4 078 075 (E. BERINGER)<br>* Seiten 0,1,6-8,10 *<br>---- | 1,11,<br>12 | C 07 D 311/56<br>A 01 N 43/16 |

RECHERCHIERTE SACHGEBIETE (Int. Cl. 3)

C 07 D 311/56
A 01 N 43/16

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 12-05-1980 | FRANCOIS |